# EUROPEAN PATENT APPLICATION

(11) **EP 4 846 939 A1**
(43) Date of publication of application: **02.12.2026**
(21) Application number: 25744589.0
(22) Date of filing: 21.01.2025
(51) Int. Cl.: A61B 5/145, A61B 5/00, G01N 33/66

(54) **CORRECTION METHOD FOR SENSITIVITY ATTENUATION OF ANALYTE SENSOR, SYSTEM AND MEDIUM**

(30) Priority: 27.01.2024 CN 202410128340
(71) Applicant: Shenzhen Sisensing Co., Ltd., Shenzhen, Guangdong 518110 (CN)
(72) Inventor: LI, Xiaobo, Shenzhen, Guangdong 518110 (CN)
(74) Representative: Vossius & Partner Patentanwälte Rechtsanwälte mbB
(86) International application number: PCT/CN2025/073766
(87) International publication number: WO 2025/157150

(57) **Abstract**

The present disclosure describes a correction method, system, and medium for sensitivity decay of an analyte sensor, the correction method including applying a first input signal to at least two electrodes of the analyte sensor at least once and acquiring a response signal corresponding to the first input signal during a correction time; obtaining a correction factor based on at least one response parameter of the response signal and at least one reference parameter of a reference signal curve; correcting a target parameter of the analyte sensor associated with the sensitivity decay based on the correction factor; determining an analyte level based on a signal associated with the analyte level acquired by the analyte sensor and a corrected target parameter. Thus, the accuracy and convenience of determining the analyte level can be improved.

## Description

### BACKGROUND OF INVENTION

The present disclosure relates to the field of biomedical engineering, and particularly to a correction method, system, and medium for sensitivity decay of an analyte sensor.

### Description of Related Art

Diabetes mellitus is a metabolic disorder involving protein, fat, water, and electrolytes that is caused by insufficient insulin secretion and reduced sensitivity of target tissue cells to insulin. Currently, continuous glucose monitoring (CGM) technology enables continuous monitoring of glucose in the user's interstitial fluid. For example, in electrochemical analysis-based continuous glucose monitoring technology, a glucose sensor used to monitor glucose concentration is generally implanted subcutaneously to monitor changes in glucose concentration in the subcutaneous interstitial fluid.

Glucose sensors used in continuous glucose monitoring typically exhibit a gradual decrease in accuracy in determining analyte levels after prolonged use, a phenomenon known as "sensitivity decay", "sensor drift", or "sensor fading." Sensitivity decay may be caused by various factors, including sensor aging, immune response, or environmental factors. Currently, a commonly used correction method for sensitivity decay involves collecting fingertip blood from the user to measure blood glucose levels, which are then used as correction values for continuous glucose monitoring.

However, repeated collection of fingertip blood reduces user comfort and convenience. How to reduce the impact of sensitivity decay on glucose detection without relying on additional blood collection requires further study.

### SUMMARY OF INVENTION

The present disclosure has been made in view of the above-mentioned state of the art, and its objective is to provide a correction method, system, and medium for sensitivity decay of an analyte sensor that improves the accuracy and convenience of determining analyte levels.

To this end, a first aspect of the present disclosure provides a correction method for correcting sensitivity decay of an analyte sensor, including applying a first input signal to at least two electrodes of the analyte sensor at least once and acquiring a response signal corresponding to the first input signal during a correction time; obtaining a correction factor based on at least one response parameter of the response signal and at least one reference parameter of a reference signal curve; correcting a target parameter of the analyte sensor associated with the sensitivity decay based on the correction factor; determining an analyte level based on a signal associated with the analyte level acquired by the analyte sensor and the corrected target parameter.

In a first aspect of the present disclosure, the electrochemical performance can be measured in real time during use of the analyte sensor by applying a first input signal to at least two electrodes of the analyte sensor and acquiring corresponding response signals during a correction time; a correction factor is obtained based on at least one response parameter of the response signal and at least one reference parameter of a reference signal curve, and by taking the reference signal curve as a standard, the correction factor enabling the response parameter to approach the reference parameter can be obtained; by correcting the target parameter based on the obtained correction factor and then determining the analyte level based on the corrected target parameter, the analyte sensor that experiences sensitivity decay can obtain the analyte level as if no sensitivity decay had occurred. Thus, the accuracy of determining the analyte level can be improved. In addition, rather than correcting the analyte sensor using fingertip blood as a standard, using the reference signal curve corresponding to the state without sensitivity decay as a standard can improve the convenience of determining the analyte level.

In addition, in the correction method according to the first aspect of the present disclosure, optionally, the signal waveform of the first input signal includes a combination of at least two pulse waves having different pulse amplitudes to form a stepwise signal waveform. In this case, the stepwise signal waveform has different pulse amplitudes, and applying signal waveforms with different pulse amplitudes to the electrodes enables the corresponding response signals to reflect more information about the electrodes, such as specificity, response time, or linear range, thereby enabling the correction factors used in correction to contain more electrode information, thus further improving the accuracy of determining the analyte level.

In addition, in the correction method according to the first aspect of the present disclosure, optionally, fitting is performed based on the response signal to obtain a response signal curve, coordinate transformation is performed on the response signal curve, and at least one response parameter of the response signal is obtained based on the response signal curve after coordinate transformation. In this case, by fitting the obtained discrete data into a continuous response signal curve, information associated with sensitivity decay can be easily extracted from the response signal; in addition, the response signal can be smoothed through fitting, thereby reducing data fluctuations caused by acquisition errors or other factors; in addition, interpolation can be performed via fitting to obtain a continuous response signal curve, thereby improving the accuracy of the obtained response parameters. In addition, coordinate transformation can convert the response signal curve from a curved form into a linear form, enabling direct processing of the straight line without considering how to select the slope of characteristic points when the response signal curve is in a curved form, thus simplifying the correction method.

In addition, in the correction method according to the first aspect of the present disclosure, optionally, the reference signal curve is obtained based on a second input signal before correction and the reference signal curve is stored, and the correction factor is obtained based on at least one response parameter of the response signal and at least one reference parameter of the reference signal curve during correction, the signal waveform of the second input signal is the same as that of the first input signal; and/or the reference signal curve is obtained based on a third input signal before correction, a plurality of decay signal curves are obtained based on the third input signal at different time periods, a target relationship is determined based on at least one reference parameter of the reference signal curve and at least one response parameter of the plurality of decay signal curves and the target relationship is stored, the correction factor is determined based on at least one response parameter of the response signal and the target relationship during correction, the target relationship represents a relationship between the response parameter and the correction factor, the signal waveform of the third input signal is the same as that of the first input signal. In this case, by performing correction based on the stored reference signal curve when using the second input signal, the integrity of the correction standard can be maintained, and information loss can be reduced compared to converting the reference signal curve into other forms of information, thereby improving the accuracy of determining the analyte level. In addition, when using the third input signal, the target relationship is obtained based on the reference signal curve and the decay signal curve before correction, and during correction, the correction factor is derived from the target relationship. This allows the correction factor to be obtained either through simple calculation or by searching a lookup table, reducing processing steps during correction, thereby improving correction speed and enhancing the real-time performance of the correction method.

**In** addition, in the correction method according to the first aspect of the present disclosure, optionally, the reference signal curve is obtained based on a preset formula associated with the input signal; and/or the reference signal curve is obtained based on a response signal corresponding to the applied input signal. In this case, obtaining the reference signal curve via the preset formula enables rapid acquisition of the reference signal curve before correction, and allows for an ideal reference signal curve without sensitivity decay-i.e., a value close to the theoretical value unaffected by sensitivity decay; in addition, by obtaining the response signal by actually applying the input signal to obtain the reference signal curve, the reference signal curve obtained before correction can be made close to the true value at which no sensitivity decay occurs.

In addition, in the correction method according to the first aspect of the present disclosure, optionally, when the input signal is a step wave, the preset formula is expressed as: $I = \frac{A}{\sqrt{t}}$, wherein I is the reference signal curve, A is a coefficient of proportionality, and t is time. Thus, the reference parameter can be derived from the coefficient of proportionality in the preset formula, and the correction factor can then be obtained based on the reference parameter.

In addition, in the correction method according to the first aspect of the present disclosure, optionally, the parameter type of the response parameter is identical to the parameter type of the reference parameter, the parameter type including one or more of peak value, area under the curve, and curve slope, or a combination thereof. In this case, it is possible to facilitate a direct comparison of the response parameter and the reference parameter, so that a difference between the response signal and the reference signal can be obtained. In this case, when the parameter type is peak value, it reflects the maximum response value of the response signal to the input signal, thereby, the maximum response capability of the analyte sensor to the input signal can be obtained, thus obtaining the degree of sensitivity decay. In addition, when the parameter type is area under the curve, it reflects the cumulative response amount of the response signal to the input signal, thereby the charge accumulation capacity of the analyte sensor can be obtained, thus obtaining the degree of sensitivity decay. In addition, when the parameter type is curve slope, it reflects the rate of rise or fall of the response signal, thereby the response speed of the analyte sensor can be obtained, thus obtaining the degree of sensitivity decay.

In addition, in the correction method according to the first aspect of the present disclosure, optionally, the target parameter is the sensitivity coefficient, and the sensitivity of the analyte sensor is corrected based on the sensitivity coefficient, with the analyte level being determined based on the corrected sensitivity; and/or the target parameter is the current compensation coefficient, and the current value of the analyte sensor is corrected based on the current compensation coefficient, with the analyte level being determined based on the corrected current value. In this case, sensitivity or current value can be indirectly corrected by adjusting the target parameter.

A second aspect of the present disclosure provides an analyte monitoring system including an analyte sensor and a processing module; the analyte sensor being configured to acquire a signal associated with an analyte level and to apply an input signal to at least two electrodes of the analyte sensor and to acquire a response signal corresponding to the input signal during a correction time; the processing module is configured to receive the signal and the response signal and to determine the analyte level using a correction method according to the first aspect of the present disclosure. Thus, the accuracy and convenience of the analyte monitoring system in determining analyte levels can be improved.

In addition, in an analyte monitoring system according to the second aspect of the present disclosure, optionally, the analyte sensor further includes a mode switching module, the mode switching module including a switching circuit configured to enable the analyte sensor to have a working mode and a correction mode. In this case, the analyte sensor can facilitate the determination of the analyte level during the working time and the correction during the correction time, thereby improving the operational efficiency of the analyte monitoring system.

A third aspect of the present disclosure provides a computer-readable storage medium storing at least one instruction which, when executed by a processor, is configured to implement the correction method according to the first aspect of the present disclosure, or to implement the analyte monitoring system according to the second aspect of the present disclosure.

According to the present disclosure, a correction method, system, and medium for sensitivity decay of an analyte sensor are provided, capable of improving the accuracy and convenience of determining analyte levels.

### BRIEF DESCRIPTION OF THE DRAWINGS

Embodiments of the present disclosure will now be explained in further detail, by way of example only, with reference to the accompanying drawings.
FIG. 1 is a diagram showing an application scenario of an analyte monitoring system according to an example of the present disclosure.
FIG. 2 is a system block diagram showing an analyte monitoring system according to an example of the present disclosure.
FIG. 3 is a flowchart showing a correction method for the sensitivity decay of an analyte sensor according to an example of the present disclosure.
FIG. 4A is a schematic diagram showing a first embodiment of a signal waveform of a first input signal according to an example of the present disclosure.
FIG. 4B is a schematic diagram showing a second embodiment of a signal waveform of a first input signal according to an example of the present disclosure.
FIG. 4C is a schematic diagram showing a third embodiment of a signal waveform of a first input signal according to an example of the present disclosure.
FIG. 4D is a schematic diagram showing a fourth embodiment of a signal waveform of a first input signal according to an example of the present disclosure.
FIG. 4E is a schematic diagram showing a fifth embodiment of a signal waveform of a first input signal according to an example of the present disclosure.
FIG. 5 is a schematic diagram showing the acquisition of a response signal according to an example of the present disclosure.
FIG. 6 is a flowchart showing the application of a first input signal and the acquisition of a corresponding response signal according to an example of the present disclosure.
FIG. 7A is a flowchart showing a first embodiment of obtaining a correction factor according to an example of the present disclosure.
FIG. 7B is a flowchart showing a second embodiment of obtaining a correction factor according to an example of the present disclosure.

### DETAILED DESCRIPTION

Hereinafter, preferred embodiments of the present disclosure will be described in detail with reference to the accompanying drawings. In the following description, like reference numerals are assigned to like components, and repeated descriptions thereof will be omitted. In addition, the drawings are only schematic, and the proportions of dimensions between components, component shapes, etc., may differ from actual implementations.

It should be noted that the terms "comprise" and "have", and any variations thereof, such as processes, methods, systems, products, or devices including or having a series of steps or units, are not necessarily limited to those steps or units expressly listed, but may include or have other steps or units not expressly listed or inherent to such processes, methods, products, or devices.

The present disclosure relates to a correction method for sensitivity decay of an analyte sensor (hereinafter may be simply referred to as a correction method, and may also be referred to as an analyte sensor correction method or a calibration method, etc.), and the correction method of the present disclosure may apply to the case where the analyte sensor is in a sensitivity decay state, and the analyte level obtained when the analyte sensor is in the sensitivity decay state deviates from the analyte level obtained when the analyte sensor is not in the sensitivity decay state, i.e., the offset analyte level is obtained. For example, due to long use time, the sensor performance degrades due to chemical reactions, physical changes, or wear, or the like, i.e., sensitivity decay occurs in the analyte sensor. The accuracy and convenience of determining analyte levels can be improved by the correction method provided in the present disclosure.

The sensitivity to which the present disclosure relates can be a parameter associated with the ability of the sensor to detect or respond to changes in the analyte level. The sensitivity coefficient to which the present disclosure relates may be a parameter for correcting the sensitivity of an analyte sensor. The sensitivity to which the present disclosure is directed may be a parameter for correcting a signal from an analyte sensor associated with the analyte level (hereinafter referred to simply as the analyte signal).

It is noted that improving the accuracy of determining an analyte level according to the present disclosure means obtaining an analyte level after correction that is close to or equal to the analyte level obtained when no sensitivity decay has occurred. For example, in cases where the analyte is glucose in interstitial fluid, the analyte level is the glucose concentration in the interstitial fluid, and the analyte sensor may be a sensor including an enzyme that undergoes a redox reaction with glucose (e.g., glucose oxidase or glucose dehydrogenase); improving the accuracy of determining the analyte level according to the present disclosure means obtaining a corrected glucose concentration that is close to or equal to the glucose concentration obtained when the analyte sensor does not experience sensitivity decay.

Improving the accuracy of determining an analyte level according to the present disclosure means obtaining an analyte level that is close to or equal to a reference analyte level. For example, when the analyte is glucose, the reference analyte level may be the glucose concentration in fingertip blood.

The present disclosure also provides an analyte monitoring system that can acquire an analyte signal, apply an input signal to two electrodes of an analyte sensor and acquire the corresponding response signal during a correction time, receive the analyte signal and the response signal, and determine the analyte level using the above-mentioned correction method. The usage duration of the analyte monitoring system may be 1 week, 2 weeks, 3 weeks, or 4 weeks. The correction method according to the present disclosure enables the analyte monitoring system to maintain good accuracy in determining analyte levels throughout its usage period.

In some examples, the analyte can be one or more of glucose, acetylcholine, amylase, bilirubin, cholesterol, chorionic gonadotropin, creatine kinase, creatine, creatinine, DNA, fructosamine, glutamine, growth hormone, hormone, ketone body, lactate, oxygen, peroxide, prostate-specific antigen, prothrombin, RNA, thyroid-stimulating hormone, or troponin. In some examples, when the analyte is glucose, the analyte level may be a glucose concentration.

The analyte sensor requiring correction in the examples of the present disclosure may be an analyte sensor experiencing sensitivity decay. An analyte sensor not requiring correction may be an analyte sensor not experiencing sensitivity decay.

Examples of the present disclosure will be described below with reference to the accompanying drawings, taking glucose in interstitial fluid as an example of the analyte, and such description does not limit the scope of the present disclosure.

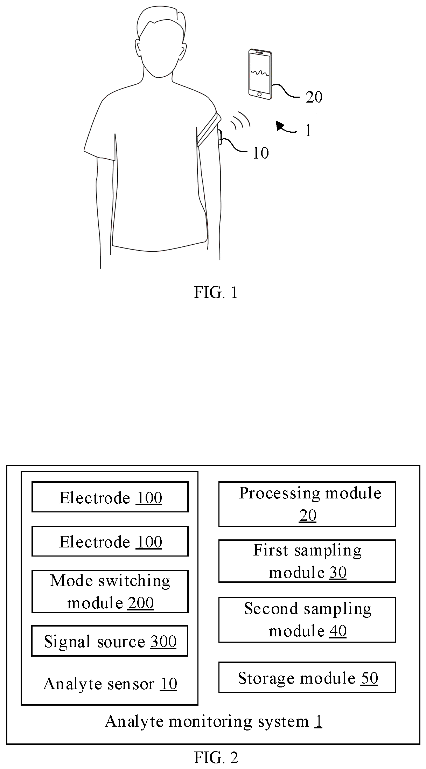

FIG. 1 is a diagram showing an application scenario of an analyte monitoring system 1 according to an example of the present disclosure.

In some examples, the analyte monitoring system 1 may include an analyte sensor 10 and a processing module 20. As shown in FIG. 1, the analyte sensor 10 may be implanted subcutaneously or mounted on the skin surface of a target subject, and the analyte sensor 10 may be configured to acquire analyte signals. The processing module 20 may be configured to receive an analyte signal and determine an analyte level. The present disclosure is not limited to the placement of the processing module 20 shown in FIG. 1, for example, the processing module 20 may also be placed on the skin surface of a target subject.

FIG. 2 is a system block diagram showing an analyte monitoring system 1 according to an example of the present disclosure.

In some examples, the analyte sensor 10 may include at least two electrodes 100. At least two electrodes 100 may be implanted subcutaneously in a subject to react with an analyte in subcutaneous tissue fluid and produce a continuous signal associated with the analyte level. At least two electrodes 100 may include a working electrode and a counter electrode. In this case, at least two electrodes 100 of the analyte sensor 10 are capable of reacting with the analyte and forming a circuit so as to generate a continuous signal associated with the analyte level.

The at least two electrodes 100 referred to in the present disclosure may also include three electrodes 100. In some examples, the three electrodes 100 may be a working electrode, a reference electrode, and a counter electrode, respectively. At least two electrodes 100 may also include four electrodes 100. In some examples, the four electrodes 100 may be a working electrode, a reference electrode, a blank electrode, and a counter electrode, respectively. In this case, the analyte signal generated can be made more accurate by using the function of the other electrodes 100, e.g., a reference electrode to provide a reference steady potential.

In some examples, the processing module 20 may determine the analyte level based on the analyte signal and the target parameter. Specifically, the processing module 20 may amplify, filter, analog-to-digital convert the signal, and calculate an analyte level corresponding to the signal based on the target parameter.

The target parameter to which the present disclosure relates may be a parameter associated with sensitivity decay. For example, the target parameter may be a sensitivity coefficient. The target parameter may also be other parameters used to determine the analyte level.

When the analyte sensor 10 is in a sensitivity decay state, the analyte monitoring system 1 may determine the analyte level using a correction method according to the present disclosure. Specifically, the correction time may be set during a gap in the working time. During the working time, the analyte monitoring system 1 may acquire an analyte signal and determine an analyte level based on the analyte signal and a target parameter. During the correction time, the analyte monitoring system 1 may apply an input signal to at least two electrodes 100 of the analyte sensor 10, acquire a response signal corresponding to the input signal, and determine an analyte level based on the analyte signal and the response signal using a correction method according to the present disclosure. Thus, the accuracy with which the analyte sensor 10 experiencing sensitivity decay determines the analyte level can be improved.

As shown in FIG. 2, the analyte monitoring system 1 may include an analyte sensor 10 and a processing module 20. The analyte sensor 10 may include at least two electrodes 100. The analyte sensor 10 may be configured to acquire an analyte signal and, during the correction time, apply an input signal to at least two electrodes 100 of the analyte sensor 10 and acquire a response signal corresponding to the input signal. The processing module 20 may be configured to receive an analyte signal and a response signal and determine an analyte level using a correction method according to the present disclosure.

In some examples, the analyte sensor 10 may also include a mode switching module 200. The mode switching module 200 may include a switching circuit to enable the analyte sensor 10 to have a working mode and a correction mode. In some examples, during a working time, the mode switching module 200 may switch to a working mode and the analyte monitoring system 1 may determine the analyte level. In some examples, during a correction time, the mode switching module 200 may switch to a correction mode and the analyte monitoring system 1 may correct the target parameter. In this case, it is possible to facilitate the analyte sensor 10 in determining the analyte level during the working time and performing correction during the correction time, thereby improving the operational efficiency of the analyte monitoring system 1.

In some examples, the analyte sensor 10 may also include a signal source 300. The signal source 300 may be configured to generate an input signal. The generated input signal may be applied to at least two electrodes 100 of the analyte sensor 10. Preferably, when the analyte sensor 10 includes three electrodes 100, it can be applied to both the working electrode and the reference electrode. The input signals may include a first input signal, a second input signal, and a third input signal. The signal waveform of the input signal may include a combination of one or more of a pulse wave, a step wave, a sawtooth wave, a triangular wave, a sine wave, and a rectangular wave. The second input signal may have the same signal waveform as the first input signal. The third input signal may have the same signal waveform as the first input signal.

In some examples, the analyte monitoring system 1 may also include a first sampling module 30. The first sampling module 30 may sample the continuous signal associated with the analyte level generated by at least two electrodes 100 during the working time to obtain a discrete signal associated with the analyte level. Specifically, the first sampling module 30 may include an analog-to-digital converter to sample the continuous signal and obtain the discrete signal.

In some examples, the first sampling module 30 may also sample the response signal corresponding to the input signal during the correction time. In some examples, the analyte monitoring system 1 may also include a second sampling module 40, the second sampling module 40 may be configured to sample the response signal corresponding to the input signal during the correction time. That is, when sampling the response signal corresponding to the input signal during the correction time, either the first sampling module 30 used for sampling during the working time or the separate second sampling module 40 may be used.

The analyte monitoring system 1, according to the present disclosure, may include at least two circuits. Namely, at least a working circuit formed by a working electrode and a counter electrode, and a measurement circuit formed by the working electrode and a reference electrode. Acquiring an analyte signal according to the present disclosure may involve acquiring the current signal flowing through the working electrode during the working time. Acquiring a response signal according to the present disclosure may involve acquiring the current signal flowing through the working electrode corresponding to the input signal. For example, a voltage is applied as a first input signal to a working electrode and a reference electrode; an enzyme disposed on the working electrode reacts with an analyte and generates a current, which is acquired as a response signal at the working electrode. The current as a response signal may be associated with the sensitivity decay of the analyte sensor 10. The current as a response signal may also be associated with the analyte level. Acquisition of the analyte signal and acquisition of the response signal may be performed by one sampling module or by two different sampling modules.

In some examples, the analyte monitoring system 1 may also include a storage module 50. The storage module 50 may store relevant data for correcting the target parameter. In some examples, the relevant data for correcting the target parameter may be a target relationship (including a look-up table or adjustment coefficient), a reference signal curve, at least one reference parameter of the reference signal curve, or a correction factor.

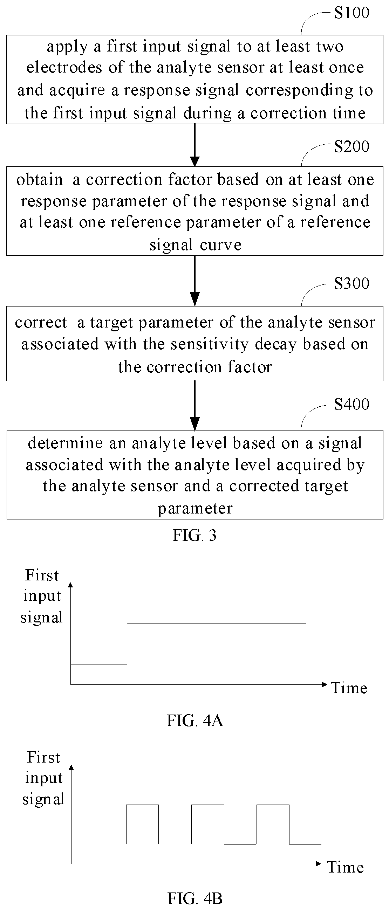

FIG. 3 is a flowchart showing a correction method for the sensitivity decay of an analyte sensor 10 according to an example of the present disclosure.

The correction method according to the present disclosure may be applied to the analyte monitoring system 1 described above. Further, the correction methods of the present disclosure may be applied to the processing module 20 described above, i.e., the processing module 20 may determine analyte levels using the correction methods of the present disclosure.

Referring to FIG. 3, in some examples, a correction method may include: a first input signal is applied to at least two electrodes 100 of the analyte sensor 10 at least once and a response signal corresponding to the first input signal is acquired during a correction time (step S100); a correction factor is obtained based on at least one response parameter of the response signal and at least one reference parameter of a reference signal curve (step S200); a target parameter associated with sensitivity decay of the analyte sensor 10 is corrected based on the correction factor (step S300); and an analyte level is determined based on a signal associated with the analyte level acquired by the analyte sensor 10 and the corrected target parameter (step S400).

With continued reference to FIG. 3, in some examples, a first input signal may be applied to at least two electrodes 100 of the analyte sensor 10 at step S100. In some examples, the signal waveform of the first input signal may include a combination of one or more of a pulse wave, a step wave, a sawtooth wave, a triangular wave, a sine wave, a staircase wave, and a rectangular wave. Combining, in the context of the present disclosure, refers to the superposition of a plurality of signal waveforms. Specifically, a plurality of signal waveforms may be aligned in period and superimposed or staggered in period and superimposed. For example, a signal waveform having one pulse wave with a higher pulse amplitude can be obtained when two pulse waves are aligned in phase and superimposed, and a signal waveform having two pulse waves can be obtained when two pulse waves are staggered in phase and superimposed. In some examples, the first input signal may be generated by the signal source 300.

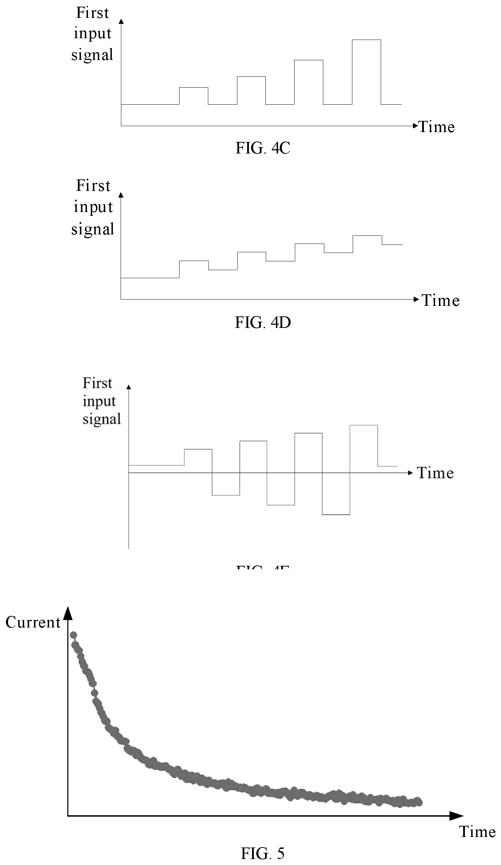

FIG. 4A is a schematic diagram showing a first embodiment of a signal waveform of a first input signal according to an example of the present disclosure. FIG. 4B is a schematic diagram showing a second embodiment of a signal waveform of a first input signal according to an example of the present disclosure. FIG. 4C is a schematic diagram showing a third embodiment of a signal waveform of a first input signal according to an example of the present disclosure. FIG. 4D is a schematic diagram showing a fourth embodiment of a signal waveform of a first input signal according to an example of the present disclosure. FIG. 4E is a schematic diagram showing a fifth embodiment of a signal waveform of a first input signal according to an example of the present disclosure.

As described above, the signal waveform of the first input signal may include a combination of one or more of a pulse wave, a step wave, a sawtooth wave, a triangular wave, a sine wave, a staircase wave, and a rectangular wave. To this end, the present disclosure also provides some examples of signal waveforms of the first input signal.

Referring to the first embodiment shown in FIG. 4A, the signal waveform of the first input signal may be a step wave. The horizontal axis represents time, and the vertical axis represents the amplitude of the first input signal. In addition, the first input signal may return to a low level or other level that does not affect the analyte monitoring system 1 operating in the working mode after the application is complete.

Referring to the second embodiment shown in FIG. 4B, the signal waveform of the first input signal may be a pulse wave. As shown in FIG. 4B, a plurality of cycles of pulse waves may be applied during the correction time. For example, 2, 3, 4, 5, 6, 7, 8, 9, or 10 cycles of the pulse wave may be applied.

In some examples, the signal waveform of the first input signal may include a combination of at least two pulse waves. Preferably, the pulse amplitudes of at least two pulse waves may be different to form a stepwise signal waveform. In this case, the stepwise signal waveform has different pulse amplitudes, and applying the signal waveform with different pulse amplitudes to the electrode 100 enables the corresponding response signal to reflect more information about the electrode 100, such as specificity, response time, or linear range, thereby enabling the correction factor used during correction to incorporate more information from the electrode 100, thus further improving the accuracy of determining the analyte level. To this end, the present disclosure also provides some examples of stepwise signal waveforms. It is noted that the present disclosure is not limited to the signal waveforms shown in FIG. 4C, FIG. 4D, and FIG. 4E, and other stepwise signal waveforms having signal gradients are within the scope of the present disclosure, such as a stepped-down signal waveform.

Referring to the third embodiment shown in FIG. 4C, the signal waveform of the first input signal may include a combination of at least two pulse waves, and form a signal waveform having a plurality of pulse waves different in pulse amplitude in a time sequence, the plurality of pulse waves different in pulse amplitude forming a stepwise signal waveform. In this case, the pulse waves having different pulse amplitudes enable different degrees of charge transfer to occur in the electrode 100, thereby enabling the kinetic characteristics of electrochemical reactions occurring in the electrode 100 under the pulse waves having different pulse amplitudes to be obtained. Thus, the obtained correction factor can be made to contain information associated with the kinetic characteristics of the electrode 100 under pulse waves having different pulse amplitudes, and correction based on the correction factor containing information associated with the dynamic characteristics of the electrode 100 can optimize the ability of the analyte sensor 10 to determine the analyte level, so that the accuracy of determining the analyte level can be further improved.

Referring to the fourth embodiment shown in FIG. 4D, the signal waveform of the first input signal may include a combination of at least two pulse waves and form a signal waveform including a plurality of pulse waves having the same pulse amplitude and a staircase wave in time sequence, the combination of the pulse waves and the staircase wave forming a stepwise signal waveform. In this case, the staircase wave included in the signal waveform of the first input signal has a relatively long duration, enabling information associated with the steady-state response of the electrode 100 to be obtained; the pulse wave included in the signal waveform of the first input signal has a relatively short duration, enabling information associated with the transient response of the electrode 100 to be obtained. Thus, the obtained correction factor can be made to contain information associated with the steady-state response and transient response of the electrode 100, and correction based on the correction factor containing information associated with the steady-state response and transient response of the electrode 100 can optimize the steady-state response capability and transient response capability of the analyte sensor 10, so that the accuracy of determining the analyte level can be further improved.

Referring to the fifth embodiment shown in FIG. 4E, the signal waveform of the first input signal may include a combination of at least two pulse waves and form a signal waveform of a plurality of pulse waves having different pulse amplitudes in time sequence, and at the same time, the directions of two adjacent pulse waves are different, i.e., the positive and negative directions alternate, and the plurality of pulse waves having different pulse amplitudes form a stepwise signal waveform. In this case, the difference in direction of the pulse waves enables information associated with the reverse reaction of the electrode 100 to be obtained. Thus, the obtained correction factor can be made to contain information associated with the reverse reaction of the electrode 100, and correction based on the correction factor containing information associated with the reverse reaction of the electrode 100 can reduce the influence of the reverse reaction on the determination of the analyte level by the analyte sensor 10, so that the accuracy of determining the analyte level can be further improved.

FIG. 5 is a schematic diagram showing the acquisition of a response signal according to an example of the present disclosure.

In some examples, a response signal corresponding to the first input signal may be acquired. As shown in FIG. 5, a response signal and its corresponding acquisition time may be obtained when acquiring the response signal. In this case, a response parameter associated with sensitivity decay can be obtained during use of the analyte sensor 10, thereby facilitating correction for sensitivity decay during operation. In some examples, the acquired response signals may be discrete data. Thus, digital processing of the response signal by means of an algorithm is facilitated.

In some examples, a fit may be performed based on the response signal to obtain a response signal curve. The response signal may be discrete data obtained by either the first sampling module 30 or the second sampling module 40 shown in FIG. 5. In this case, by fitting the discrete data obtained by the first sampling module 30 or the second sampling module 40 into a continuous response signal curve, it can be convenient to extract information associated with the sensitivity decay from the response signal; in addition, the response signal can be smoothed through fitting, thereby reducing data fluctuations caused by acquisition errors or other factors; in addition, interpolation can be performed via fitting to obtain a continuous response signal curve, thereby improving the accuracy of the obtained response parameters.

In some examples, the response signal curve obtained by the fitting may include a rising segment and/or a falling segment. For example, the acquired response signal shown in FIG. 5 may be fitted to obtain a response signal curve that includes a descending segment.

In some examples, the response signal curve may not exclude a straight line. That is, the response signal curve may include a curve form and a straight-line form. The discrete response signals can be fitted directly to a curve form.

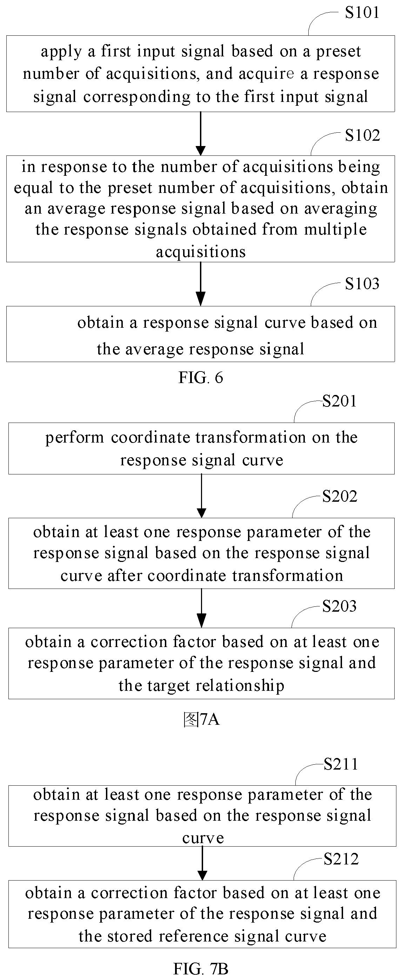

FIG. 6 is a flowchart showing the application of a first input signal and the acquisition of a corresponding response signal according to an example of the present disclosure.

As described above, in step S100, a first input signal may be applied to at least two electrodes 100 of the analyte sensor 10 at least once and a response signal corresponding to the first input signal is acquired during the correction time. The number of times the first input signal is applied to at least two electrodes 100 of the analyte sensor 10 during the correction time may be one, two, three, four, five, six, seven, eight, nine, or ten times.

Referring to FIG. 6, in some examples, applying a first input signal and acquiring a corresponding response signal may include: applying a first input signal based on a preset number of acquisitions, and acquiring a response signal corresponding to the first input signal (step S101); in response to the number of acquisitions being equal to the preset number of acquisitions, obtaining an average response signal based on averaging the response signals obtained from multiple acquisitions (step S102); and obtaining a response signal curve based on the average response signal (step S103).

With continued reference to FIG. 6, in some examples, in step S101, a first input signal may be applied based on a preset number of acquisitions. The first input signal can be applied multiple times during the correction time, and the number of times of applying the first input signal can be a preset number of acquisitions. In addition, the first input signal may return to a low level or other level that causes the response signal to return to a steady state when the first input signal is not applied during the gaps between multiple applications. The time required for the gaps between multiple applications may be sufficient to restore the response signal to a steady state when the first input signal is not applied. In this case, it is possible to reduce mutual interference between a plurality of response signals generated by applying an input signal multiple times, and thus to improve the accuracy of obtaining response parameters. The number of times the first input signal is applied may be associated with a preset number of acquisitions.

With continued reference to FIG. 6, in some examples, in step S102, in response to the number of acquisitions being equal to a preset number of acquisitions, an average response signal may be obtained based on averaging response signals obtained from multiple acquisitions. Specifically, a first input signal is applied, and a response signal is acquired for a corresponding number of times, and the response signals are averaged to obtain an average response signal. The number of times the first input signal is applied is equal to the number of times the response signal is acquired, i.e., equal to the preset number of acquisitions. In this case, the accuracy of the acquired response signal can be improved by acquiring the response signal a plurality of times and averaging it to obtain an average response signal.

With continued reference to FIG. 6, in some examples, in step S103, a response signal curve may be obtained based on the average response signal. In this case, by obtaining the response signal curve from the average response signal, the accuracy of at least one response parameter of the response signal obtained based on the response signal curve can be improved, thereby improving the accuracy of correction based on the response parameter.

Referring back to FIG. 3, in some examples, in step S200, a correction factor may be obtained based on at least one response parameter of the response signal and at least one reference parameter of the reference signal curve. In addition, the reference signal curve may represent the response signal curve of the response signal corresponding to the input signal when no sensitivity decay occurs in the analyte sensor 10. In this case, by using the reference signal curve corresponding to the absence of sensitivity decay as a standard, a correction factor that brings the response parameter closer to the reference parameter can be obtained. In addition, using the reference signal curve corresponding to the state where the analyte sensor 10 does not experience sensitivity decay as the standard can reduce the influence of uncertain factors on the accuracy of the standard, compared with using signal curves obtained when the analyte sensor 10 is in other states. In addition, unlike correcting the analyte sensor 10 using fingertip blood as a reference, using the reference signal curve corresponding to the absence of sensitivity decay improves the convenience of determining the analyte level.

With continued reference to FIG. 3, in some examples, in step S200, at least one response parameter of the response signal may be obtained based on a response signal curve determined from the response signal. The parameter type of the response parameter may include one or more of a peak value, an area under the curve, and a curve slope. In this case, when the parameter type is a peak value, it reflects the maximum response value of the response signal to the input signal, thereby the maximum response capability of the analyte sensor 10 to the input signal can be obtained, thus obtaining the degree of sensitivity decay; in addition, when the parameter type is the area under the curve, it reflects the cumulative response amount of the response signal to the input signal, thereby the charge accumulation ability of the analyte sensor 10 is obtained, thus obtaining the degree of sensitivity decay; in addition, when the parameter type is the curve slope, it reflects the rate of increase or decrease of the response signal, thereby the response speed of the analyte sensor 10 is obtained, thus obtaining the degree of sensitivity decay.

In some examples, the response signal curve may be in the form of a curve. The peak in the parameter type may be the peak value of the response signal curve. The area under the curve in the parameter type may be the integral over time of the response signal curve. The curve slope in the parameter type may be the slope of the curve in the target segment. In addition, the target segment may be a descending segment or an ascending segment. Wherein the descending segment can correspond to the response signal obtained from acquisition in FIG. 5. In some examples, the curve slope in the parameter type may be the median of the slopes in the target segment, the slope at half-peak (i.e., at half the peak value) of the response signal curve within the target segment, or the slope corresponding to a characteristic point on the target segment.

In some examples, the response signal curve may be in the form of a straight line. The curve slope in the parameter type may be the slope of the straight line. A response signal curve in the form of a straight line (described later) can be obtained by performing a coordinate transformation on the response signal curve in the form of a curve. In this case, the slope can be directly obtained from the straight line without considering how to select the slope when the response signal curve is curved, thereby simplifying the correction method.

In some examples, the reference signal curve may be obtained based on the response signal corresponding to the applied input signal. Specifically, an input signal may be applied to an analyte sensor 10 that does not experience sensitivity decay, a response signal may be acquired, and at least one reference parameter of the response signal may be obtained. That is, the reference signal curve is the corresponding response signal obtained by applying an input signal to the analyte sensor 10, where no sensitivity decay occurs. In this case, by obtaining the response signal through actual application of the input signal to acquire the reference signal curve, the reference signal curve obtained before correction can be made close to the true value in the absence of sensitivity decay.

In some examples, the applied input signal may be the second input signal. In some examples, a reference signal curve may be obtained based on the second input signal, and the reference signal curve may be stored before correction. Specifically, the second input signal may be configured to be applied to the analyte sensor 10 outside of the correction time without requiring correction. The signal waveform of the second input signal may be the same as that of the first input signal. The time outside the correction time referred to in the present disclosure may be a working period within the target object's use period, or a factory production or assembly period prior to the target object's use period, etc.

In some examples, the second input signal may be generated by the signal source 300 when the analyte monitoring system 1 is in use. In some examples, the second input signal may be generated by an external signal source prior to use of the analyte monitoring system 1. In some examples, the external signal source may be a function signal generator. In some examples, a reference signal corresponding to the second input signal may be acquired. Fitting may be performed based on the reference signal to obtain a reference signal curve.

In some examples, the reference signal curve obtained based on the second input signal may be stored in the storage module 50. In this case, the reference signal curve is obtained and saved by applying the second input signal, enabling the analyte monitoring system 1 to determine a correction factor based on the saved reference signal curve during the correction time.

In some examples, the applied input signal may be the third input signal. In some examples, a reference signal curve can be obtained based on the third input signal before correction. The third input signal may be configured to be applied to the sample sensor without correction outside of the correction time and to be applied to the sample sensor requiring correction during different time periods. The sample sensor may be a sensor used in obtaining the target relationship. The sample sensor may simulate the situation in which the analyte sensor 10 experiences different degrees of sensitivity decay and the situation in which no sensitivity decay occurs. The third input signal may have the same signal waveform as the first input signal. The third input signal may be generated by an external signal source. In some examples, the reference signal curve obtained based on the third input signal may be stored in the storage module 50. In this case, the correction factor can be determined based on the stored reference signal curve of the sample sensor during the correction time, without applying an input signal to the analyte sensor 10 before the correction time, so that the acquisition cost of the reference signal curve can be reduced and the lifetime of the analyte sensor 10 can be maintained. Thus, the accuracy with which analyte sensor 10 determines analyte levels can be further improved.

In some examples, a reference signal corresponding to the third input signal may be acquired. Fitting may be performed based on the reference signal to obtain a reference signal curve.

In some examples, the third input signal may also be used to obtain a decay signal curve, which may be used to determine a target relationship (described later). Specifically, a plurality of decay signal curves may be obtained based on the third input signal at different time periods. The different time periods may be respective time periods corresponding to different degrees of sensitivity decay of the sample sensor.

In some examples, a response signal corresponding to the third input signal may be acquired. A fit may be performed based on the response signal to obtain a decay signal curve.

With continued reference to FIG. 3, in some examples, in step S200, a reference signal curve may be obtained based on a preset formula associated with the input signal. In this case, the reference signal curve is obtained by a preset formula, so that the reference signal curve can be obtained quickly before correction, and an ideal reference signal curve without sensitivity decay can be obtained, i.e., a theoretical value close to the case without sensitivity decay can be obtained. In some examples, when the input signal is a step wave or a pulse wave, the preset formula is expressed as:
$I = \frac{A}{\sqrt{t}} ,$
wherein, I is the reference signal curve, A is the coefficient of proportionality, and t is time. Thus, the reference parameter can be derived from the coefficient of proportionality in the preset formula, and the correction factor can then be obtained based on the reference parameter.

With continued reference to FIG. 3, in some examples, at least one reference parameter of a reference signal curve may be acquired in step S200. When the input signal is a step wave or a pulse wave, the preset formula is $I = \frac{A}{\sqrt{t}}$, such that, in order to keep at least one response parameter of the response signal identical to at least one reference parameter of the reference signal curve for comparison, the reference signal curve having the preset formula can be subjected to a coordinate transformation, transforming the reference signal curve from a curve form into a straight-line form, and the curve slope among the at least one reference parameter can be A.

In some examples, the parameter type of the reference parameter and the parameter type of the response parameter may be identical. For example, if the parameter type of the response parameter includes a peak value, the parameter type of the reference parameter may also include a peak value. In this case, it is possible to facilitate a direct comparison of the response parameter and the reference parameter, so that a difference between the response signal and the reference signal can be obtained.

With continued reference to FIG. 3, in some examples, in step S200, a correction factor may be obtained during correction based on at least one response parameter of the response signal and a stored reference signal curve. Specifically, at least one response parameter of the response signal and at least one reference parameter of the stored reference signal curve may be analyzed to obtain a difference between the response signal and the reference signal curve, and the correction factor is obtained based on the difference. In addition, the stored reference signal curve may be a reference signal curve obtained based on the second input signal, the third input signal, or a preset formula described above.

In some examples, a correction factor may be obtained based on the difference between the response signal and the reference signal curve. In some examples, the difference between the response signal and the reference signal curve may be the difference between the response parameter and the reference parameter. For example, the difference or ratio between the response parameter and the reference parameter, etc. In some examples, the correction factor may be obtained based on a difference or ratio between the response parameter and the reference parameter. For example, the ratio between the response parameter and the reference parameter may be used as a correction factor.

In other examples, different correction factors may be used to correct the target parameter of the analyte sensor 10, and the correction results may be compared to obtain a correction factor. The target parameter of the analyte sensor 10 is corrected using different correction factors. For example, the different correction factors may be 0.7, 0.8, 0.9, 1.0, 1.1, 1.2, and 1.3. Different correction factors are multiplied by the target parameter to correct the target parameter. After the target parameter of the analyte sensor 10 is corrected, the corresponding analyte level is also corrected. Using the analyte level corresponding to the stored reference signal curve as a standard, the proximity of the corrected analyte level to the analyte level corresponding to the stored reference signal curve is compared. The correction factor corresponding to the closest corrected analyte level is obtained.

In some examples, the response signal may also be aligned in time with a stored reference signal curve prior to analysis to obtain the difference. For example, alignment is performed at the start time point of the pulse wave. In this case, it is possible to improve the accuracy of analyzing the difference between the response signal and the stored reference signal curve.

With continued reference to FIG. 3, in some examples, in step S200, a correction factor may be obtained during correction based on at least one response parameter of the response signal and a target relationship. In addition, the target relationship may represent a relationship between the response parameter and the correction factor. Specifically, the correction factor corresponding to the response parameter may be determined based on the target relationship to obtain the correction factor. The response parameter may be at least one response parameter of a response signal corresponding to the first input signal.

In some examples, the target relationship may be a functional relationship. Further, the target relationship may be a linear relationship. When the target relationship is a linear relationship, the target relationship may be expressed by an adjustment coefficient. The adjustment coefficient may be a linear coefficient satisfying a linear relationship between the response parameter and the correction factor. In this case, by directly using an adjustment coefficient to represent the linear relationship between the response parameter and the correction factor, it is possible to reduce the amount of storage used in the storage module 50 when storing the target relationship.

Specifically, the response parameter may be multiplied by an adjustment coefficient to obtain the correction factor. For example, when the response parameter is the curve slope, the curve slope may be multiplied by an adjustment coefficient to obtain the correction factor.

In some examples, the target relationship may be represented by a look-up table. The response parameters and correction factors may be stored in a look-up table. The look-up table may represent a correspondence between the response parameter and the correction factor. In this case, it is possible to easily retrieve the response parameter to quickly obtain the corresponding correction factor.

As described above, the reference signal curve and the plurality of decay signal curves may be obtained based on the third input signal. In some examples, the target relationship may be determined based on at least one reference parameter of a reference signal curve determined from the third input signal and at least one response parameter of the plurality of decay signal curves.

In some examples, the decay signal curve may be in the form of a straight line. The curve slope in the parameter type may be the slope of the straight line. In this case, the slope can be directly obtained from the straight line without considering how to select the slope when the decay signal curve is curved, thereby obtaining parameters associated with the severity of sensitivity decay in each time period corresponding to different degrees of sensitivity decay of the sample sensor.

In some examples, different adjustment parameters may be multiplied by the response parameters to obtain different correction factors for establishing the target relationship. The target parameters of the sample sensor are corrected using different correction factors. After the target parameter of the sample sensor is corrected, the corresponding analyte level is also corrected. Using the analyte level corresponding to the reference signal curve as the standard, compare the proximity between the corrected analyte level and the analyte level corresponding to the reference signal curve. The adjustment parameter corresponding to the closest corrected analyte level is used as the target relationship.

In some examples, different correction factors may also be used to correct the sample sensor to obtain the target relationship. The target parameters of the sample sensor are corrected using different correction factors. After the target parameter of the sample sensor is corrected, the corresponding analyte level is also corrected. For example, when the target parameter is a sensitivity coefficient, after the sensitivity coefficient is corrected, the corresponding analyte level obtained based on the calculation of the target parameter is also corrected. Using the analyte level corresponding to the reference signal curve as the standard, compare the proximity between the corrected analyte level and the analyte level corresponding to the reference signal curve. The correction factor corresponding to the closest corrected analyte level and the initial response parameter is stored in a look-up table. A look-up table is used as the target relationship.

In other examples, the target relationship may be obtained by machine learning. In some examples, machine learning may involve learning at least one response parameter of the response signal and at least one reference parameter of the reference signal curve to obtain a target relationship between the response parameter and the correction factor.

In some examples, the target relationship may be obtained by at least one of an in vitro experiment and a human body-worn experiment. The in vitro experiment may involve measuring data associated with the analyte sensor 10 in a glucose solution that mimics interstitial fluid. The body-worn experiment may involve measuring data associated with the analyte sensor 10 when implanted in the interstitial fluid of a human body.

In some examples, the target relationship may be saved. In some examples, the target relationship may be stored in the storage module 50.

With continued reference to FIG. 3, in some examples, in step S200, a correction may be performed in response to the analyte sensor 10 being in a sensitivity decay state. Specifically, it may be determined that the analyte sensor 10 is in a sensitivity decay state based on a difference between at least one response parameter of the response signal and at least one reference parameter of the reference signal curve. Further, a correction may be performed in response to the analyte sensor 10 being in a sensitivity decay state. In this case, detecting that the analyte sensor 10 is in a sensitivity decay state and performing a correction enables the analyte monitoring system 1 to maintain accuracy in determining the analyte level during use with the target object.

With continued reference to FIG. 3, in some examples, in step S200, a difference between at least one response parameter and at least one reference parameter may be determined. Further, the analyte sensor 10 may be determined to be in a sensitivity decay state in response to the difference being greater than a first threshold. The first threshold may indicate that the analyte sensor 10 is in a sensitivity decay state.

As described above, the response signal curve, the reference signal curve, or the decay signal curve may take the form of a straight line. To this end, the present disclosure also provides an example of determining a response parameter or a reference parameter in the form of a straight line.

In some examples, coordinate transformation may be applied to the response signal curve, the reference signal curve, or the decay signal curve. The coordinate transformation may involve transforming the time variable of the curve so that the curve is converted from a curved form to a straight-line form. For example, when the input signal is a step wave or a pulse wave, the curve satisfies a relationship where the signal is proportional to the inverse of the arithmetic square root of time, and the time variable of the curve can be transformed from t to 1/sqrt(t), i.e., from t to the inverse of the square root of t. When the curve satisfies a relationship where the signal is proportional to the logarithm of time, the time variable of the curve can be transformed from t to ln(t). In this case, coordinate transformation enables the curve to be converted from a curved form to a straight-line form, thereby allowing direct processing of the straight line, such as calculating its slope, without having to address the difficulty of selecting feature points for slope calculation when the curve is in a curved form, thus simplifying the correction method. It should be noted that the present disclosure is not limited to the above examples, and other coordinate transformations that convert the curve from a curved form to a straight-line form are also within the scope of the present disclosure, such as combinations of the two coordinate transformations mentioned above.

In some examples, at least one response parameter of the response signal or at least one reference parameter of the reference signal may be obtained based on a response signal curve after coordinate transformation, a reference signal curve, or a decay signal curve. The curve slope may be used as a response parameter or a reference parameter for a response signal curve, a reference signal curve, or a decay signal curve. For example, when the third input signal is a step wave or a pulse wave, the time of the decay signal curve may be transformed from t to 1/sqrt(t), and the decay signal curve after coordinate transformation is transformed from a straight-line form to a curved line form. The slope of the decay signal curve after coordinate transformation may be taken as the curve slope of the decay signal curve. The curve slope of the decay signal curve may be taken as the response parameter of the decay signal curve.

FIG. 7A is a flowchart showing a first embodiment of obtaining a correction factor according to an example of the present disclosure.

As described above, in step S200, a correction factor may be obtained based on at least one response parameter of the response signal and at least one reference parameter of the reference signal curve.

Referring to FIG. 7A, in some examples, a first embodiment of obtaining a correction factor may include: performing coordinate transformation on the response signal curve (step S201), obtaining at least one response parameter of the response signal based on the response signal curve after coordinate transformation (step S202), and obtaining a correction factor based on at least one response parameter of the response signal and the target relationship (step S203).

With continued reference to FIG. 7A, in some examples, in step S201, a coordinate transformation may be performed on the response signal curve. The response signal curve may be transformed from a curvilinear form to a rectilinear form through coordinate transformation. In this case, when the input signal has a certain specific signal waveform, for example, when the input signal includes a step wave or a pulse wave, such coordinate transformation can transform the response signal curve into a straight line, thereby allowing direct processing of the straight line, such as calculating the slope, without considering how to select the slope at characteristic points when the response signal curve is curved, thus simplifying the correction method.

With continued reference to FIG. 7A, in some examples, in step S202, at least one response parameter of the response signal may be obtained based on the response signal curve after coordinate transformation. For example, when the input signal has characteristics of a signal waveform of a pulse wave or a step wave waveform, the curve slope corresponding to the response signal curve after coordinate transformation may be used as at least one response parameter of the response signal.

With continued reference to FIG. 7A, in some examples, in step S203, a correction factor may be obtained based on at least one response parameter of the response signal and the target relationship. For example, when the target relationship is a look-up table, a correction factor corresponding to the response parameter may be retrieved from the look-up table.

With continued reference to FIG. 7A, in some examples, in step S203, a target relationship may be determined and stored based on the reference signal curve and the decay signal curve corresponding to the third input signal before correction. That is, the reference signal curve may be obtained based on the third input signal before correction. A plurality of decay signal curves is obtained based on the third input signal at different time periods. The target relationship is determined based on at least one reference parameter of the reference signal curve and at least one response parameter of the plurality of decay signal curves, and the target relationship is stored. During correction, a correction factor is determined based on at least one response parameter of the response signal and the target relationship. The target relationship represents the relationship between the response parameter and the correction factor. In this case, by obtaining the target relationship based on the reference signal curve and the decay signal curve, and performing correction based on the saved target relationship, the usage amount in the storage module 50 can be reduced compared to saving the reference signal curve; in addition, the correction factor is obtained based on the target relationship during correction, the correction factor being obtained by simple calculation (for example, the response parameter multiplied by an adjustment coefficient) or by searching a look-up table, thereby simplifying the processing steps during correction, further improving the correction speed, and thus enhancing the real-time performance of the correction method.

FIG. 7B is a flowchart showing a second embodiment of obtaining a correction factor according to an example of the present disclosure.

Referring to FIG. 7B, in some examples, a second embodiment of obtaining a correction factor may include: obtaining at least one response parameter of the response signal based on the response signal curve (step S211), and obtaining a correction factor based on at least one response parameter of the response signal and the stored reference signal curve (step S212).

With continued reference to FIG. 7B, in some examples, in step S211, the parameter type of response parameter may include one or more of a peak value, area under the curve, and curve slope, or a combination thereof. When the response signal curve is in the form of a curve, the curve slope of the response signal may be the median of the slope in the descending or ascending segment, the slope corresponding to half of the peak value, or the slope corresponding to another characteristic point on the curve (e.g., the point on the curve where the slope is maximum).

With continued reference to FIG. 7B, in some examples, in step S212, the response signal may be aligned in time with the stored reference signal curve. For example, alignment may be performed at the start time point of the pulse wave, and then at least one response parameter of the response signal and at least one reference parameter of the stored reference signal curve are analyzed to obtain a difference between the response signal and the reference signal curve, and the correction factor is derived based on this difference. For example, machine learning methods can be used to analyze the differences.

With continued reference to FIG. 7B, in some examples, in step S212, the reference signal curve may be obtained based on the second input signal and stored before correction. That is, the reference signal curve may be obtained based on the second input signal and saved before correction. At the time of correction, the correction factor is obtained based on at least one response parameter of the response signal and at least one reference parameter of the reference signal curve. In this case, by performing correction based on the stored reference signal curve, the integrity of the standard used for correction can be maintained; compared to converting the reference signal curve into other forms of information, this approach reduces information loss, thereby improving the accuracy of determining the analyte level. In addition, storing the reference signal curve before correction simplifies the preparation steps before correction without requiring additional processing. In addition, when the reference signal curve is relatively complex, it is difficult to establish a mathematical relationship between the reference signal curve and the response signal curve; directly using the reference signal curve for correction improves the applicability of the correction method.

With continued reference to FIG. 3, in some examples, in step S300, a target parameter associated with the sensitivity decay of the analyte sensor 10 may be corrected based on the correction factor. The target parameter may be associated with the conversion relationship between the analyte signal and the analyte level. In some examples, when the correction factor is associated with the ratio of the response parameter to the reference parameter, the target parameter may be multiplied or divided by the correction factor for correction. In some examples, when the correction factor is associated with the difference between the response parameter and the reference parameter, the correction factor may represent a relative error, and the target parameter may be adjusted by adding or subtracting a change amount corresponding to the relative error. The change amount corresponding to the relative error may be equal to the relative error multiplied by the target parameter. In addition, the relative error may be in the form of a percentage of the difference.

In addition, the conversion relationship may be determined by the analyte sensor 10 itself. In some examples, the conversion relationship may be associated with the analyte signal and sensitivity (e.g., the analyte level may be calculated by dividing the analyte signal by the sensitivity of the analyte sensor 10), and the corrected target parameter may be used to correct the analyte signal or sensitivity. Specifically, the target parameter may be a sensitivity coefficient or a current compensation coefficient.

With continued reference to FIG. 3, in some examples, in step S400, the sensitivity of the analyte sensor 10 may be corrected based on a sensitivity coefficient, or the current value of the analyte sensor 10 may be corrected based on a current compensation coefficient. In some examples, the analyte level may be determined based on the analyte signal acquired by the analyte sensor 10 and the corrected target parameter.

With continued reference to FIG. 3, in some examples, in step S400, the analyte level may be determined based on the analyte signal acquired by the analyte sensor 10 and the corrected target parameter. In this case, by correcting the target parameter based on the obtained correction factor and determining the analyte level based on the corrected target parameter, the analyte sensor 10 experiencing sensitivity decay can obtain an analyte level as if no sensitivity decay had occurred. Thus, the accuracy with which the analyte sensor 10 experiencing sensitivity decay determines the analyte level can be improved.

With continued reference to FIG. 3, in some examples, in step S400, when the target parameter is a sensitivity coefficient, the sensitivity of the analyte sensor 10 may be corrected based on the sensitivity coefficient. In some examples, the analyte level is determined based on the analyte signal acquired by the analyte sensor 10 and the corrected sensitivity. In this case, the sensitivity can be indirectly corrected by correcting the sensitivity coefficient.

In some examples, when the target parameter is a current compensation coefficient, the current value of the analyte sensor 10 may be corrected based on the current compensation coefficient. In some examples, the analyte level is determined based on the analyte signal acquired by the analyte sensor 10 and the corrected current value. In this case, the current value can be indirectly corrected by correcting the current compensation coefficient.

One or more steps of the correction method according to the present disclosure may be performed during the correction time, such as applying the first input signal in step S100. Part of the parameters required for one or more steps of the correction method according to the present disclosure may be obtained outside the correction time, for example, during operating time while the target object is in use, or during factory production or assembly time before use of the target object; for example, at least one reference parameter, lookup table, or adjustment coefficient associated with the reference signal curve in step S200 may be obtained outside the correction time.

At least one reference parameter of the reference signal curve according to the present disclosure may be obtained from an analyte sensor 10 that does not experience sensitivity decay. In addition, the reference parameter can also be obtained after performing a correction on the analyte sensor 10 when the target object is in a stable state, such as when the target object is fasting or when the body of the target object is static during the night.

The analyte sensor 10 according to the present disclosure that does not experience sensitivity decay may be an analyte sensor 10 not in a state of sensitivity decay, such as an analyte sensor 10 during factory production or assembly time before the target object uses the analyte monitoring system 1, an analyte sensor 10 for which the difference between a response parameter and a reference parameter is less than a first threshold, an analyte sensor 10 before use or after a period of use by the target object, or a corrected analyte sensor 10.

The correction frequency of the correction method according to the present disclosure may be once per day, twice per day, once every two days, once per week, or performed in response to the analyte sensor 10 being in a state of sensitivity decay.

The dynamic properties to which the present disclosure relates include information associated with sensitivity.

The present disclosure also relates to a computer-readable storage medium that may store at least one instruction, which, when executed by a processor, implements one or more steps of the correction method described above, or implements the analyte monitoring system 1 described above.

The present disclosure also relates to an electronic device that may include a processor and a memory, wherein the processor executes a program stored in the memory to implement one or more steps of the correction method described above, or to implement the analyte monitoring system 1 described above.

While the present disclosure has been particularly shown and described with reference to the accompanying drawings and embodiments, it is to be understood that the above description is not intended to limit the present disclosure in any manner. Those skilled in the art can make various modifications and variations to the present disclosure as needed without departing from the spirit and scope of the present disclosure, and such modifications and variations fall within the scope of the present disclosure.

## Claims

1. A correction method for sensitivity decay of an analyte sensor, comprising:
applying a first input signal to at least two electrodes of the analyte sensor at least once and acquiring a response signal corresponding to the first input signal during a correction time;
obtaining a correction factor based on at least one response parameter of the response signal and at least one reference parameter of a reference signal curve;
correcting a target parameter of the analyte sensor associated with the sensitivity decay based on the correction factor;
determining an analyte level based on a signal associated with the analyte level acquired by the analyte sensor and a corrected target parameter.

2. The correction method for sensitivity decay according to claim 1, wherein,
a signal waveform of the first input signal comprises a combination of at least two pulse waves having different pulse amplitudes to form a stepwise signal waveform.

3. The correction method for sensitivity decay according to claim 1, wherein,
fitting is performed based on the response signal to obtain a response signal curve, coordinate transformation is performed on the response signal curve, and at least one response parameter of the response signal is obtained based on the response signal curve after coordinate transformation.

4. The correction method for sensitivity decay according to claim 1, wherein,
the reference signal curve is obtained based on a second input signal before correction and the reference signal curve is stored, and the correction factor is obtained based on at least one response parameter of the response signal and at least one reference parameter of the reference signal curve during correction, the signal waveform of the second input signal is the same as that of the first input signal; and/or
the reference signal curve is obtained based on a third input signal before correction, a plurality of decay signal curves are obtained based on the third input signal at different time periods, a target relationship is determined based on at least one reference parameter of the reference signal curve and at least one response parameter of the plurality of decay signal curves and the target relationship is stored, the correction factor is determined based on at least one response parameter of the response signal and the target relationship during correction, the target relationship represents a relationship between the response parameter and the correction factor, the signal waveform of the third input signal is the same as that of the first input signal.

5. The correction method for sensitivity decay according to claim 1, wherein,
the reference signal curve is obtained based on a preset formula associated with an input signal; and/or
the reference signal curve is obtained based on a response signal corresponding to an applied input signal.

6. The correction method for sensitivity decay according to claim 5, wherein, when the input signal is a step wave, the preset formula is expressed as: $I = \frac{A}{\sqrt{t}} ,$ wherein I is the reference signal curve, A is a coefficient of proportionality, and t is time.

7. The correction method for sensitivity decay according to claim 1, wherein,
the parameter type of the response parameter is identical to the parameter type of the reference parameter, the parameter type comprises one or more of peak value, area under the curve, and curve slope, or a combination thereof.

8. The correction method for sensitivity decay according to claim 1, wherein,
the target parameter is a sensitivity coefficient; the sensitivity of the analyte sensor is corrected based on the sensitivity coefficient, and the analyte level is determined based on corrected sensitivity; and/or
the target parameter is a current compensation coefficient; the current value of the analyte sensor is corrected based on the current compensation coefficient, and the analyte level is determined based on a corrected current value.

9. An analyte monitoring system, comprising an analyte sensor and a processing module;
the analyte sensor being configured to acquire a signal associated with an analyte level and to apply an input signal to at least two electrodes of the analyte sensor and to acquire a response signal corresponding to the input signal during a correction time;
the processing module being configured to receive the signal and the response signal and to determine the analyte level using the correction method according to any one of claims 1 to 8.

10. The analyte monitoring system according to claim 9, wherein,
the analyte sensor further comprises a mode switching module, the mode switching module comprises a switching circuit to enable the analyte sensor to have a working mode and a correction mode.

11. A computer-readable storage medium, wherein,
the computer-readable storage medium stores at least one instruction which, when executed by a processor, implements the correction method according to any one of claims 1 to 8, or implements the analyte monitoring system according to any one of claims 9 to 10.
